(19) Europäisches Patentamt
European Patent Office
Office européen des brevets

(11) Publication number: **0 167 337**
**A2**

# (12) EUROPEAN PATENT APPLICATION

(21) Application number: 85304507.8

(51) Int. Cl.[4]: C 11 D 1/14

(22) Date of filing: 25.06.85

(30) Priority: 25.06.84 US 624323
25.06.84 US 624322
25.06.84 US 624324

(43) Date of publication of application:
08.01.86 Bulletin 86/2

(84) Designated Contracting States:
BE DE FR GB IT NL

(71) Applicant: ATLANTIC RICHFIELD COMPANY
515 South Flower Street
Los Angeles California 90071(US)

(72) Inventor: Kesling, Haven S., Jr.
248 Friendship Road
Drexel Hill, PA(US)

(72) Inventor: Gillman, Hyman D.
R.D. No. 1 Box 213B
Spring City, PA(US)

(74) Representative: Cropp, John Anthony David et al,
MATHYS & SQUIRE 10 Fleet Street
London, EC4Y 1AY(GB)

## (54) Alkoxylated ether sulfate anionic surfactants.

(57) Novel alkoxylated ether sulfate anionic surfactants are prepared by oxyalkylation with propylene oxide or 1,2-butylene oxide of a straight chain alcohol having about 4 to 10 carbon atoms or a branched chain alcohol having about 4 to 11 carbon atoms or a mixture of straight and/or branched chain alcohols each having about 4 to 11 carbon atoms; optionally followed by oxyalkylation with ethylene oxide or with a mixture of ethylene oxide and higher alkylene oxide; sulfation of the alkoxylated product; and neutralisation of the sulfated derivative. The alkoxylated ether sulfate anionic surfactants of the present invention are liquids which exhibit superior detergency to polyester fabrics, excellent hard water stability, organic solubility, low foaming, low odour, and exhibit excellent compatibility with non-ionic and other surfactants in detergent formulations.

EP 0 167 337 A2

Croydon Printing Company Ltd.

# ALKOXYLATED ETHER SULFATE ANIONIC SURFACTANTS

This invention relates to certain novel alkoxylated ether sulfate anionic surfactants based on plasticiser range linear or branched alcohols to mixtures thereof and to concentrated aqueous surfactant compositions based on such mixtures. More particularly this invention relates to certain novel relatively short chain ether alcohol sulfate anionic surfactants produced by propoxylation or butoxylation of "plasticiser" range primary or branched "oxo" alcohols, optionally followed by alkoxylation with ethylene oxide or with a mixture of ethylene oxide and propylene oxide or butylene oxide, followed by sulfation and neutralisation. The surfactants of the invention exhibit properties comparable to or better than commercial surfactants prepared from "detergent" range alcohols.

During the last several years, primary "detergent" range alcohol ethoxylated sulfates have been used extensively in large volume surfactant applications such as light duty liquid dishwashing detergents, concentrated laundry detergents, hard surface cleaners, and as textile surfactants. The advantages of the ethoxylated ether sulfates over the previously employed alcohol sulfates include ready synthesis, increased solubility, and insensitivity to hard water (see for example T.P. Matson, Soap and Chemical Specialties, November 1963).

Propoxylated and butoxylated alcohol ether sulfate anionic surfactants have been disclosed in the prior art, but are not known to have been employed in commercial applications. A substantial portion of prior art disclosing such surfactant compositions deals only with "detergent" or fatty range alcohols, i.e. $C_{12}$-$C_{18}$ alcohols, as disclosed, for example, in Weil et al. U.S. Patent 4,383,706, and with short oxypropylene chains generally containing between about 1 and 3 propylene oxide units, as disclosed, for example in Tuvell et al U.S. Patent 3,775,349. Although certain ether alcohol sulfate compositions derived from straight chain carbon alcohols of shorter chain length and containing greater than 3 oxypropylene units have been studied, for example, by J. Chlebicki et al., "Synthesis and Surface Activity of Sodium Polyoxypropylated Higher Alcohol Sulfates", Tenside Detergents, 17, 130-134 (1980), the authors

conclude that these materials are generally inferior surfactants as compared with propoxylated surfactants prepared from "detergent" range alcohols.

Fabric softeners employable in the laundry wash, rinse or dryer cycle are desired for commercial application. Furthermore, because of the inconvenience of rinse and dryer cycle application, the industry is attempting to develop softeners that are compatible with wash cycle applications. Cationic quaternary ammonium salts, which are used commercially in fabric softening applications, cannot be used in the wash cycle with anionic surfactants. It is believed that the cationic and the anionic materials complex and precipitate, thus reducing detergency. Although wash cycle detergent/fabric softner formulations have been prepared from non-ionic surfactants and cationic softners, these formulations lack the detergent power that can be obtained when an anionic surfactant is used as the detergent.

In one of its aspects, the present invention provides new and useful anionic surfactants based on plasticiser range linear alcohols or branched oxo alcohols,i.e. relatively short chain, as compared with detergent alcohols.

Said anionic surfactants yield water- and organic solvent-soluble compositions characterised as being low foaming and free of unpleasant odour, and which exhibit excellent detergency, particularly with respect to use of polyester fabrics, and excellent stability in hard water. The surfactants may also form non-viscous, readily flowable, liquid, highly active concentrated anionic surfactant compositions requiring no added solubilising agent.

At least the surfactants of the present invention based on branched oxo alcohols are highly active liquid anionic surfactant compositions employable without the use of other solvents, other than water.

Thus, in accordance with one aspect of the present invention there is provided an anionic surfactant composition corresponding to the formula:

$$RO[CH_2CH(R')O]_mZ_nSO_3M \qquad \text{(Formula I)}$$

wherein

R is a primary hydrocarbon alkyl radical containing from about 4 to 10 carbon atoms or a branched chain hydrocarbon alkyl radical containing from about 4 to 11 carbon atoms preferably 7 to 10 carbon atoms;

R' is a member selected from the group consisting of methyl and ethyl; m is an integer which is from 7 to 12, preferably 8 to 10, when R is a primary hydrocarbon alkyl radical and from 5 to 12, preferably 6 to 10, when R is a branched chain hydrocarbon alkyl radical;

Z is an oxyethylene group or a random mixture of oxyethylene groups and oxyalkylene groups present in the radical $-[CH_2CH(R')O]-$, the molar ratio of oxyethylene to oxyalkylene groups in said mixture being such that the total molar ratio of oxyethylene to oxyalkylene groups in said formula is from about 1:1 to 1:10; n is an integer of from 0 to 4; and M is hydrogen, an alkali metal, an alkaline earth metal; ammonium or a primary, secondary, tertiary or quaternary alkyl ammonium or alkylolammonium ion.

Illustrative alkoxylated ether sulfate anionic surfactants of the present invention include:

where R is linear:

$$n\text{-}C_8H_{17}O(CH_2\overset{CH_3}{C}HO)_7SO_3Na$$

$$n\text{-}C_8H_{17}O(CH_2\overset{CH_3}{C}HO)_7(CH_2CH_2O)_3SO_3Na$$

$$n\text{-}C_8H_{17}O(CH_2\overset{CH_3}{C}HO)_{10}(CH_2CH_2O)_3SO_3Na$$

$$n\text{-}C_8H_{17}O(CH_2\overset{\overset{CH_3}{CH_2}}{C}HO)(CH_2\overset{CH_3}{C}HO)_7(CH_2CH_2O)_3SO_3Na$$

$$n\text{-}C_8H_{17}O(CH_2\overset{\overset{CH_3}{CH_2}}{C}HO)(CH_2\overset{CH_3}{C}HO)_7(CH_2CH_2O)_2SO_3Na$$

$$n\text{-}C_8H_{17}O(CH_2\overset{\overset{CH_3}{CH_2}}{CHO})_2(CH_2\overset{CH_3}{CHO})_7SO_3Na$$

$$n\text{-}C_8H_{17}O(CH_2\overset{CH_3}{CHO})_8SO_3K$$

$$n\text{-}C_8H_{17}O(CH_2\overset{CH_3}{CHO})_8SO_3NH_4$$

$$n\text{-}C_8H_{17}O(CH_2\overset{CH_3}{CHO})_8SO_3N(CH_2CH_2OH)H_3$$

$$n\text{-}C_8H_{17}O(CH_2\overset{CH_3}{CHO})_8SO_3N(CH_3)_4$$

$$n\text{-}C_8H_{17}O(CH_2\overset{CH_3}{CHO})_8SO_3N(CH_3)_2(CH_2CH_2OH)H$$

$$n\text{-}C_{10}H_{21}O(CH_2\overset{CH_3}{CHO})_9SO_3Na$$

$$n\text{-}C_{10}H_{21}O(CH_2\overset{CH_3}{CHO})_{10}SO_3Na$$

where R is branched:

$$CH_3CH_2CH_2CH_2CH(CH_2CH_3)CH_2O(CH_2\overset{CH_3}{CHO})_{10}\ SO_3Na$$

$$CH_3CH_2CH_2CH_2CH(CH_2CH_3)CH_2O(CH_2\overset{CH_3}{CHO})_{10}\ (CH_2CH_2O)_2SO_3Na$$

$$CH_3CH_2CH_2CH_2CH(CH_2CH_3)CH_2O(CH_2\overset{\overset{CH_3}{CH_2}}{CHO})_2\ (CH_2\overset{CH_3}{CHO})_8SO_3Na$$

$$CH_3CH_2CH_2CH_2CH(CH_2CH_3)CH_2O(CH_2\overset{CH_3}{CHO})_{12}\ (CH_2CH_2O)_2SO_3Na$$

$$CH_3CH_2CH_2CH_2CH(CH_2CH_3)CH_2O(CH_2\overset{\overset{CH_3}{CH_2}}{CHO})_2\ (CH_2\overset{CH_3}{CHO})_8\ (CH_2CH_2O)_2SO_3Na$$

$$CH_3CH_2CH_2CH_2CH(CH_2CH_3)CH_2O(CH_2\overset{CH_3}{CHO})_{10}\ SO_3NH_4$$

$$CH_3CH_2CH_2CH_2CH(CH_2CH_3)CH_2O(CH_2\overset{CH_3}{CHO})_{10}\ SO_3\ N(CH_3)_4$$

$$CH_3CH_2CH_2CH_2CH(CH_2CH_3)CH_2O(CH_2\overset{CH_3}{CHO})_{10}\ SO_3\ N(CH_2CH_2OH)_2(CH_3)_2$$

$$CH_3CH_2CH_2CH_2CH(CH_2CH_3)CH_2O(CH_2\overset{CH_3}{CHO})_{10}\ SO_3\ N(CH_2CH_2OH)_4$$

$$CH_3CH_2CH_2CH_2CH(CH_2CH_3)CH_2O(CH_2\overset{CH_3}{CHO})_{10}\ SO_3\ K$$

$$CH_3CH_2CH_2CH_2CH(CH_2CH_3)CH_2O(CH_2\overset{CH_3}{CHO})_{10}\ SO_3\ N(CH_2CH_2OH)_3H$$

$$i\text{-}C_5H_9O(CH_2\overset{CH_3}{CHO})_{12}SO_3Na$$

$$i\text{-}C_9H_{19}O(CH_2\overset{CH_3}{CHO})_{10}SO_3Na$$

In accordance with the present invention, liquid low foaming alkoxylated ether sulfate anionic surfactant compositions based on linear or branched chain alcohols having superior detergency characteristics for textile materials and fabric softening properties are provided.

The invention also provides mixtures of different alkoxylated ether sulfate anionic surfactants.

Mixtures of surfactants have previously been prepared and sold for a wide variety of industrial and domestic applications. Such compositions often are required in fluid form, and it is desirable that they should contain as high a proportion of active material as possible, in order to reduce the cost of storage and transport. However, in the case of most surfactant mixtures, it has generally been impossible to obtain a fluid composition at concentrations above about 30 to 50 percent and higher, by weight, of active ingredient, and further, depending upon the nature of the mixture, small amounts of water, i.e. up to about 10 percent may depress the melting point slightly while larger quantities of water result in the formation of a rigid gel rather than a fluid solution as a result of a phase change. Hence, it has generally been found that as the total concentration of surface active ingredient in a dilute solution approaches a critical level, which usually ranges between about 30 to 50 percent by weight or higher, the viscosity of the solution begins to rise, causing difficulty in preparing and handling

the solution. At the critical level the solution sets into an immobile gel or phase separation occurs.

It has sometimes been possible to increase the concentration of active ingredient by addition of viscosity modifiers or co-solvents, such as alcohols, which act as thinners thereby lowering the viscosity of the solution and inhibiting the formation of gels, and permitting the obtainment of higher concentrate solutions. However, such co-solvents are normally effective in producing substantial increases in the attainable concentration when they are present in readily large quantities, which solvents, at these concentrations, constitute a fire hazard and may adversely affect the properties of the product compositions for many of their desired end uses and/or increase the cost of the product.

As used herein, the term "active concentration" will be used to denote the total concentration of "active" i.e. surface active, ingredients in the aqueous anionic surfactant composition.

Aqueous solutions of sulfates of alkoxylated fatty alcohols, generally containing from 10 to 24 carbon atoms in the alcohol chain, such as sulfated ethoxylated lauryl alcohol, or mixtures containing lauryl alcohol derivatives, have been employed in cosmetic, toiletry and other detergent compositions. In general, these compositions are supplied at about 30 percent active ingredient, but in the interest of economy and transport and packaging, high concentrations of the order of 50 to 70 percent, are also commercially available. At these high concentrations they have the texture of a thick paste. In the final formulation, these compositions are normally present in an amount of less than about 30 percent active ingredients in aqueous solution. As indicated above, unfortunately, upon dilution of these concentrates with water, rather than the viscosity diminishing as might be expected, the viscosity begins to substantially increase; for example, the sodium salt of the sulfate of a di-ethoxylated derivative of a commonly used mixture of alcohols containing between 10 and 18 carbon atoms having a concentration of 60% active matter, the balance being water, has a viscosity of approximately 17,000 centipoises. Upon dilution to 45% active matter, the viscosity increases to greater than 500,000 centipoises. Upon further dilution,

the viscosity drops until at the concentration used in shampoos, for example, it again becomes liquid.

Although it has been possible in some instances to employ such concentrates without the incorporation of viscosity modifiers, such an increase in the viscosity during dilution naturally present substantial problems for the formulator. Such problems include the formation of lumps and gels which are themselves only difficultly soluble on attempted further dissolution. In order to avoid these problems, viscosity modifiers have been incorporated, as above indicated, in aqueous concentrates of sulfates of ethoxylated fatty alcohol so as to maintain viscosity of the solution at high concentrations at a level such that the solutions are reasonably free-flowing and are easily diluted through any required concentration. In addition to modifying the viscosity of these solutions, it is also necessary that any additive employed should not prevent or hinder the effect of thickeners conventionally used, such as sodium or ammonium salts, which may be incorporated in the finished formulation in order to provide an acceptable consistency. At present, this problem is being reduced by incorporating into the concentrated aqueous sulfate of ethoxylated fatty alcohol, an alcohol of low molecular weight such as isopropranol or ethanol. However, the use of such alcohols is disadvantageous in that having high vapour pressure their odour is detectable in the finished formulation and, further, such compositions may constitute a fire hazard as above indicated.

In accordance with a further aspect, the present invention provides aqueous solutions of admixtures of anionic surfactants based on plasticiser range linear or branched alcohols.

The anionic surfactant admixtures provide water and organic solvent-soluble anionic surfactant admixture compositions characterised as being free of unpleasant odour and exhibiting excellent detergency and stability in hard water and low foaming properties and may be provided in the form of highly active concentrated anionic surfactant, free-flowing admixture compositions requiring no added solubilising agents and which are capable of being diluted with water without formation of undesired highly viscous lumps or gel phase.

In accordance with this aspect of the invention there is provided an alkoxylated ether sulfate anionic surfactant admixture composition corresponding to the formula:

$$RO[CH_2CH(R')O]_mZ_nSO_3M \qquad \text{(Formula II)}$$

wherein R is a straight or branched chain hydrocarbon alkyl radical containing from about 4 to 11 carbon atoms; R' is a member selected from the group consisting of methyl and ethyl; m is an integer of from 5 to 12, preferably 6 to 10; Z is an oxyethylene group or a random mixture of oxyethylene groups and oxyalkylene groups present in the radical $-[CH_2CH(R')O]-$, the molar ratio of oxyethylene to oxyalkylene groups in said mixture being such that the total molar ratio of oxyethylene to oxyalkylene groups in said formula is from about 1:1 to 1:10; n is an integer of from 0 to 4; and M is hydrogen, an alkali metal, an alkaline earth metal; ammonia or a primary, secondary, tertiary or quaternary alkyl ammonium or alkylolammonium ion, said admixture being characterised as containing at least two of said surfactants present in a mole ratio between about 4:1 and 1:4.

The alkoxylated ether sulfate anionic surfactant components may be straight or branch-chain, depending upon the precursor alcohol employed in preparation of the surfactant composition.

Preferred components of the admixture compositions of the invention conform to the above-identified formula wherein R is a straight chain alkyl radical containing from about 8 to 10 carbon atoms; when R' is methyl, m is up to 9, and when R' is ethyl, m is up to 6, n is 0, and M is an alkali metal, such as sodium or potassium, or ammonium. It is to be understood that additional components conforming to Formula II above may also be present as part of the surfactant admixture composition, and when present, generally constitute up to ten percent by weight of the composition. In addition, the surfactant compositions of the invention may also contain minor amounts, up to about two percent, each by weight, of alkoxylated sulfates of $C_4$ or $C_{12}$ alcohols.

It has been surprisingly found that these admixtures of alkoxylated ether sulfate anionic surfactants based on plasticiser range linear

and/or branched oxo-alcohols exhibit unexpectedly improved detergency characteristics for textile materials and fabric softening properties, as compared with the individual alkoxylated alcohols, or with commercially available alkoxylated fatty alcohol derived surfactants.

Illustrative of alkoxylated ether sulfate anionic surfactants which may serve as components of the admixture compositions of the present invention are those listed above as specific examples of individual alkoxylated ether surfactants wherein R is a primary hydrocarbon radical and as specific examples of such surfactants wherein R is a branched chain hydrocarbon radical.

The anionic surfactant compositions of the present invention (whether comprising individual alkoxylated ether sulfates or admixtures thereof) have been found to exhibit important advantages as detergent intermediates compared to alkoxylated anionic surfactant compositions produced from ethylene oxide alone. Hence, it has been found that the physical properties of the alkoxylated ether sulfates of the invention contrast with properties of commercially available ethoxylated ether sulfates derived from "detergent" or fatty range alcohols, which are solids at concentrations of greater than about 60%, whereas the alkoxylated ether sulfate surfactants of the invention are liquids at concentrations of 85% (or 90% in the case of the mixtures of alkoxylated ether sulfates) or greater. In addition, the ethoxylated ether sulfates derived from fatty alcohols require a solvent, e.g. 10 weight percent ethanol, in order to maintain a solution even at the 50 to 60 weight percent concentration; in contrast, no solvent except water is required to dissolve or stabilise the individual or admixed alkoxylated ether sulfate surfactants of the present invention. The alkoxylated ether sulfate surfactants of the present invention will also avoid a gel phase upon dilution with water. These differences in physical properties provide the surfactants of the present invention with a significant economic advantage associated with shipping and storage and in end use applications.

In addition at least the individual alkoxylated ether sulfates of the invention can be spray dried without pluming and without the flammability problems associated with using ethanol in

ethoxylated anionic surfactant formulations.

Furthermore, the alkoxylated alcohol ether sulfates and alkoxylated alcohol ether sulfate mixtures of the invention are equivalent to or better as detergents than commercially employed surfactant compositions, for example dodecylbenzene sulfonate compositions, non-ionic surfactants, and commercially available ethoxylated ether sulfates, such as Neodol® 25-3S. Moreover, the alkoxylated ether sulfates and alkoxylated ether sulfate mixtures of the invention are substantive to cotton and are employable as wash cycle fabric softeners; results obtained are comparable with those obtained using commercially available wash cycle fabric softening formulations.

Moreover, the alkoxylated ether sulfates and alkoxylated ether sulfate mixtures of the present invention are stable and exhibit good detergency at hard water hardness of 500 ppm or higher and also have been shown to be exceptional lime soap dispersants, as compared with commercially available dodecylbenzene sulfonate compositions. Other advantages characteristic of the surfactant compositions of the present invention include good primary biodegradability, excellent solubility and good detergency in both hot and cold water, excellent solubility in organic solvents such as toluene, hexane, and 1,1,1-trichloroethane, non-corrosion to mild steel and most polymers, ability to be spray dried without volatility loss, good alkaline stability, and excellent compatibility with non-ionic and other surfactants in detergent formulations. The alkoxylated ether sulfate surfactants of the present invention, while being anionic in character, have many of the desirable properties of a nonionic surfactant.

The concentrated aqueous surfactant compositions of the invention comprise at least about 5%, but no more than about 50%, generally not more than about 40% by weight of water and an alkoxylated ether sulfate anionic surfactant conforming to Formula I above or an active mixture comprising at least two of the alkoxylated ether sulfate anionic surfactants conforming to Formula II above, present in a ratio of between about 1:4 and 4:1, preferably between about 1:2 and 2:1. These compositions are capable of dilution with water to any concentration of active ingredient without formation of a gel phase or lumps.

In accordance with the present invention, it has been further found that aqueous solutions of the alkoxylated ether sulfate anionic surfactant present in a wide range of "active concentrations" may be prepared; these solutions exhibit viscosity and dilution properties that enable them to be prepared as concentrates and diluted with water when preparing a detergent formulation containing "active concentration" of surfactant conventionally employed in such formulations, without detracting from the properties of such formulations, i.e. are readily diluted with water to any required or desired concentration without forming gels or lumps and are reasonably free-flowing. Hence, such solutions are, in general, characterised by having a viscosity, at 25°C, of less than 1000 cps, and preferably, of less than about 100 cps. As used herein, the term "active concentration" is used to denote the total concentration of "active" i.e. surface active, ingredients in the aqueous anionic surfactant composition.

The surfactant compositions and admixtures of the invention may be prepared by known methods, for example, by alkoxylation of the

appropriate individual linear or branched chain alcohol or likewise by alkoxylation of an admixture of alcohols. In the case where an individual alcohol is used, it is to be understood that the term linear alcohol means an alcohol containing from 4 to 10 carbon atoms in the chain of which substantial quantities i.e. about 95% or greater are primary. Likewise, the term branched chain alcohol means an alcohol containing 4 to 11 carbon atoms in the chain of which substantial quantities, i.e. about 50% or greater, preferably 80% or greater, are branched.

Alkoxylation of the alcohol or alcohol admixture may be effected, for example, at elevated temperatures, generally between about 70° and 150°C, preferably between about 90°C and 100°C at pressures ranging from atmospheric to about 500 psig, preferably between about 50 and 100 psig, in the presence of an alkaline catalyst, such as an alkali metal hydroxide, illustratively, potassium hydroxide, present in a concentration ranging from between about 0.01 and 1 weight percent preferably between about 0.2 and 0.3 weight percent of the reactant. In general, a controlled amount of propylene oxide or 1,2-butylene oxide, or an admixture thereof, is slowly contacted with the alcohol or alcohol admixture, which optionally may be preheated to a liquid state, over a reaction time, generally ranging up to about 20 hours, in an amount sufficient to form the desired oxyalkylated reaction product mixture.

In general, sufficient alkylene oxide is employed in the alkoxylation reaction to effect preparation of an alkoxylated derivative having an average number of alkylene oxide units per molecule in the surfactant product which is between 7 and 12, and preferably between 8 and 10, where R is a primary hydrocarbon radical; between 5 and 12, preferably between 6 and 10, when R is a branched chain hydrocarbon radical and between 5 and 12, preferably between 6 and 10, where an alcohol admixture is used. Optionally, if desired, in a second step, as is known in the art, for example, from Martin J. Schick, "Nonionic Surfactants", pp. 118, Marcel Dekker, Inc., New York (1966) ethylene oxide may be added to the reaction product of the first alkoxylation step to produce an ethylene oxide "tipped" product having a primary hydroxyl group, or alternatively, this second alkoxylation step may be effected by use of a mixture of ethylene oxide and propylene oxide or 1,2-butylene oxide, thereby producing an alkoxylated ether characterised by a block or random

structure, under the reaction conditions specified above for the first alkoxylation reaction. In general, when ethylene oxide alone is added, approximately one to four moles of ethylene oxide is added, and when a mixture of ethylene oxide and propylene oxide or 1,2-butylene oxide is employed, the molar ratio of ethylene oxide to such higher alkylene oxide range from about 1:1, preferably from about 2:1 to about 5:1. The amount of oxides employed when the second alkoxylation is performed is such that the resultant product contains a total molar ratio of oxyethylene groups to oxypropylene or oxybutylene groups from about 1:1 to about 1:10, and preferably from about 1:2 to about 1:4.

If desired, the oxyalkylation of the alcohol or alcohol admixture may be carried out in a suitable solvent, illustratively, an aromatic hydrocarbon such as toluene, benzene, ethers such as tetrahydrofuran and the like. Other solvents employable for this purpose include aliphatic hydrocarbons containing from about 5 to 12 carbon atoms, such as heptane, hexane, octane and the like, thereby obviating the toxic associations connected with use of aromatic hydrocarbon solvents, if employed. It is also necessary to ensure that the alcohol reactant is free of water, and hence, vacuum stripping of the starting material may be employed in conventional manner.

Primary alcohols which may be employed in the preparation of the alkoxylated intermediates in production of the surfactant compositions of the present invention are commercially available and may be obtained, for example, by the Ziegler process, as disclosed in F. Asinger,

"Mono-Olefin Chemistry and Technology" Pergamon Press, NW (1968). Illustrative suitable alcohols include n-butanol; n-hexanol; n-heptanol; n-octanol; n-nonanol and n-decanol.

Branched chain alcohols which may be employed in the preparation of the alkoxylated intermediates in production of the surfactant compositions of the present invention are commercially available and may be obtained, for example, by the oxo process, as disclosed in Richard F. Heck, "Organotransition Metal Chemistry" Academic Press, New York (1974). Illustrative suitable alcohols include 3-methyl butanol, 2-ethyl propanol, isopropanol, 2-pentanol, 2-hexanol, 2-ethylhexanol, 2-nonanol, iso-nonanol and 2-ethylnonanol.

Alcohols suitable for use where admixtures are employed include those given above as examples of primary and branched chain alcohols. Isomeric oxo nonanol admixtures may also be mentioned.

The alkoxylated alcohols or alcohol admixtures obtained are converted to the sulfates typically by reaction with chlorosulfonic acid, sulfur trioxide, or concentrated sulfuric acid in accordance with well known procedures such as disclosed in U.S. Patents 2,187,244 and 3,931,271. In general, for example, when sulfation is effected by use of chlorosulfonic acid, a slight excess of chlorosulfonic acid diluted with equal portion volumes of a solvent such as methylene chloride, is slowly added to a solution of oxyalkylated oligomer, present in equal volume portion, in methylene chloride over a period of 2 hours. The temperature is held at 10° to 25°C using an icebath and nitrogen sweep. Sulfation with chlorosulfonic acid in the absence of solvent is also possible if good control over heat transfer is maintained.

The surfactant compositions of the invention are of high quality characterised as being essentially free of unpleasant odour. This is a significant advantage as compared with those alkoxylated surfactant compositions produced from Ziegler type alcohols which have been alkoxylated solely by ethylene oxide. It has been reported that such compositions derived from Ziegler alcohols exhibit a high and objectionable odour level. In contrast, the use of propoxylation or butoxylation in production

of the desired surfactant compositions of the invention eliminates the undesired odour associated with alkoxylated surfactants which are solely ethoxylated products. This observation is rationalised by considering the relative reactivity of ethylene oxide and higher alkylene oxides such as propylene oxide or butylene oxide with Ziegler alcohols; in the case of ethylene oxide alone, the product ether alcohol from ethoxylation is a primary alcohol, whereas, in the case of said higher alkylene oxides, the product is a secondary ether alcohol which is considerably less likely to react relative to the starting alcohol or impurities which are primary alcohols. Thus, all the starting alcohol impurities are alkoxylated, e.g. propoxylated, before further propoxylation of the secondary alcohol product occurs, and hence, propoxylation of these impurities significantly decreases volatility and thus, reduces odour.

According to the invention it is not necessary to separate the individual alkylene oxide ether alcohols by distillation or other separation techniques in order to accomplish the objectives of the invention. The entire reaction mixture which will contain an average number of alkylene oxide units selected from propylene oxide or 1,2-butylene oxide, or optionally, in addition thereto a random mixture of such oxyalkylene groups with oxyethylene groups, may be directly sulfated to provide an anionic surfactant product with improved solubility, exhibiting the physical and chemical properties referred to hereinabove.

Following sulfation, the alkoxylated ether sulfate composition corresponding to Formula I or II, above wherein M is hydrogen, is neutralized by known methods, for example, by reaction with: an alkali metal hydroxide, such as sodium hydroxide, as the most preferred, potassium hydroxide, lithium hydroxide; an alkaline earth metal hydroxide such as magnesium hydroxide, calcium hydroxide, cesium hydroxide, rubidium hydroxide, or aluminum hydroxide; ammonia or a substituted amine derivative thereof. Typical substituted amine derivative reactants employable for this purpose include ammonia, triethanolamine, triisopropanolamine and the like. Normally the neutralization agent is employed in concentration ranging from between about 25 and 50 weight percent to produce a product with a pH of between about 7 and 10, preferably between 7 and 7.7. If desired, a solvent such as a lower alkanol, illustratively ethanol or isopropanol in a concentration of at least 10%, when employed, may be used.

The following examples will serve to illustrate the practice of the invention, but they are not intended to limit it to the details described herein. Parts and percentages are by weight, temperatures are in degrees Centigrade, unless otherwise specified.

## EXAMPLES

A. Preparation of alkoxylated derivative of appropriate alcohol.

All reactions were carried out in a 2-gallon oil heated stirred stainless steel autoclave. A 0.25 percent catalyst solution was prepared using the appropriate "plasticiser" Ziegler or oxo alcohol or alcohol admixture and potassium hydroxide by preheating at 75 to 100°C. Water was removed under vacuum and the catalyst solution was charged hot under dry nitrogen into the autoclave. The remaining alcohol was added to the reactor followed by purging with dry nitrogen. The reactor was heated to 95° and dry alkylene oxide in the appropriate stoichiometry was slowly added over a period of 24-48 hours using a pressure demand control valve system to control the addition rate. A reference pressure was set at 60 psig, and if the reactor pressure dropped below this pressure the control valve opened and more alkylene oxide was charged to the reactor. When the pressure increased to greater than 60 psig, the valve closed. The alkylene oxide was contained in a hoke that was suspended on a weight load cell, thereby permitting the charging of the correct amount of alkylene oxide. Since the hoke had a 80 psig nitrogen pressure head, the overall reactor pressure increased to 80 psig when all the liquid alkylene oxide was pushed out of the

load cell hoke into the reactor. When the reaction was complete, the product was removed hot from the reactor and was treated with Magnesol® (4 grams per 250 grams of product) for 2 hours at 120° in order to remove the catalyst. The resulting product was vacuum filtered through a Cellite bed at 60-80° to provide the pure oligomeric polyol. Hydroxyl number, VPO molecular weight, GPC analysis, and $^{13}C$ NMR were primarily used to characterise the product oligomers.

B. Preparation of Sulfate as a Derivative

1. Sulfation with Chlorosulfonic Acid and Neutralization

A slight excess of chlorosulfonic acid diluted 50/50 with methylene chloride was slowly added to a solution of oxyalkylated oligomer (50/50) in methylene chloride over the course of two hours. The temperature was held at 10-25° using an ice-bath and a nitrogen sweep (approximately 2-5 liters/minute) was used to remove hydrochloric acid from the reaction zone. After the addition was complete, the mixture was allowed to stir an additional 1-2 hours at 35-40°. The excess methylene chloride solvent was stripped from the sulfated product via rotary evaporation prior to neutralization. The product was neutralized with 50 percent sodium hydroxide to a pH of 7.5-9.0, and the neutralized product was cooled overnight at 10°C and filtered to remove sodium chloride and sodium sulfate. The product was extracted with petroleum ether to remove unreacted oligomer and was stripped to remove water and any remaining solvent. Both of the above purification steps (i.e., filtration

and extraction) are optional and need not be always used. The final alkoxylated ether sulfate anionic surfactant, or surfactant admixture, product is a liquid at active concentration of 90 wt. % or greater.

2. Sulfation with Sulfur Trioxide and Neutralization

Liquid sulfur trioxide diluted with an air stream was slowly added over a two hour period at 25° in an amount sufficient to provide 65% propoxylated oligomer conversion to the appropriate alkoxylated, e.g. propoxylated polyol in methylene chloride solvent. By holding the conversion low, acid build up and unsaturation due to elimination could be prevented. The sulfur trioxide incorporated into the $SO_3Na$ containing surfactant was determined by titration with Hyamine®-1622 (to methylene blue end-point) and the unreacted sulfur trioxide was determined by scrubbing through sodium hydroxide followed by simple acid-base titration. At the end of the sulfation run, the temperature was increased to 35° for one hour and air was bubbled through the solution to ensure that all the unreacted sulfur trioxide was removed or reacted. The product was neutralized with 50 percent sodium hydroxide to a pH of 8.0 followed by extraction with petroleum ether to recover the unreacted propoxylated polyol for recycle.

Examples 1-7

A number of surfactants of the invention of the kind wherein R is a primary hydrocarbon radical were prepared using the synthetic procedures outlined in paragraphs A and B-1 above. Their properties are set forth in Table I below.

TABLE I

Properties of Alkoxylated Alcohol Sodium Sulfates

| Example No. | Alcohol | Alkylene Oxide/ Units | Surface Tension(2) (Dynes/cm) | Draves Wetting(3) (Sec) | Ross Miles Foaming (cm)(4) | | Detergency(5) Polyester | |
|---|---|---|---|---|---|---|---|---|
| | | | | | Initial | 5 Minutes | % SR | % D |
| 1 | N-Octanol | Propylene oxide/ 8 | 34.2 | 31.0 | 15.9 | 1.8 | 75.0 | 68.3 |
| 2 | N-heptanol | Propylene oxide/ 5 | 39.2 | 29.2 | 18.7 | 61 | 53.1 | 50.1 |
| 3 | N-Octanol | Propylene oxide/ 4 | 37.2 | 360.0 | 20.0 | 0.5 | 50.4 | 39.7 |
| 4 | N-Octanol | Propylene oxide/ 5 | 38.1 | 210.0 | 20.1 | 1.2 | 66.2 | 41.9 |
| 5 | Neodol®25(1) | Ethylene oxide/ 3 | 34.2 | 18.0 | 20.0 | 18.7 | 58.7 | 55.6 |
| 6 | Neodol®25(1) | Propylene oxide/ 3 | 33.2 | 17.4 | 18.4 | 15.7 | 47.5 | 45.1 |
| 7 | Neodol®25(1) | Propylene oxide/ 5 | 33.7 | 18.3 | 18.8 | 15.1 | 46.9 | 44.8 |

Notes:

(1) Neodol®25 = Shell $C_{12-15}$ fatty alcohol
(2) Measured on a 1% solution at 25°C; Kraft Points is 1°.
(3) Draves wetting is measured on a 0.1 wt% solution at room temperature (77°F)
(4) ASTM D1173-53 was used.
(5) Terg-o-tometer test with: Speed = 125 rpms; Temperature = 120°F; Time = 10 minutes/ Surfactant concentration = .15 wt%; hardness = 150 ppm; 4" x 6" cloth - each wash batch used 5 soiled and 3 clean polyester fabric; the panels used in determining evalutation were coated with standard pad applied airborne/dust sebum soil.

$$\text{\% Soil Removal (SR)} = \frac{R_L \text{ Soil BW} \times 100}{R_L \text{ Unsoiled} - R_L \text{ Soil BW}}$$

$$\text{\% Detergency (D)} = \frac{R_L \text{ Soil Aw} - (R_L \text{ Redeposition BW-AW})}{R_L \text{ Redeposition} - R_L \text{ Soil BW}} \times 1$$

$R_L$ = Reflectance (L Scale)

AW = After Wash

BW = Before Wash

The results set forth in Table I comparing a typical surfactant of the present invention (Example 1) with a commercially available surfactant (Example 5), as well as with a number of surfactants which have been ethoxylated or propoxylated to a lesser degree than that of the composition of Example 1, demonstrate the obtainment of improved results of the compositions of the present invention with regard to surface activity, wetting, foaming and as a detergent with respect to polyester fabric.

Examples 8-11

The experimental procedure outlined under Examples No. 1-7 was followed to prepare alkoxylated anionic derivatives from the alcohols and alkylene oxide indicated in Table II below; the detergency results, obtained in the same manner as in Table I, above, employing water of the hardness indicated, are set forth in Table II, below.

TABLE II

Effect Water Hardness

| Example No. | Alcohol | Alkylene Oxide (Units) | (PPM Hardness | Detergency Polyester %SR | %I |
|---|---|---|---|---|---|
| 8 | Neodol®25 | ethylene oxide(3) | 150 | 58.7 | 55.6 |
| 9 | Neodol®25 | ethylene oxide(3) | 500 | 49.1 | 47.3 |
| 10 | N-Octanol | propylene oxide(8) | 150 | 75.0 | 68.3 |
| 11 | N-Octanol | propylene oxide(8) | 500 | 75.5 | 69.4 |

The results set forth above in Table II show that water hardness has little or no effect on the propoxylated ether sulfate detergency characteristics of the invention and were comparable to those achieved by commercially available detergents.

Examples 12-17

The experimental procedure outlined under Example 1 was followed to prepare alkoxylated derivatives of the invention, while varying the base employed for neutralization; to demonstrate the counter-ion effect upon surface active properties; the detergency was obtained in the same manner as in Table I, above. The results are set forth in Table III, below.

TABLE III

| Example No. | Alcohol(R) | Alkylene Oxide/ Units | Counter-ion (M+) | Detergency Polyester %SR | %D |
|---|---|---|---|---|---|
| 12 | N-Octanol | propylene oxide/8 | Na | 75.0 | 68.3 |
| 13 | N-Octanol | propylene oxide/8 | $NH_4$ | 80.5 | 77.8 |
| 14 | N-Octanol | propylene oxide/8 | K | 76.8 | 69.9 |
| 15 | N-Octanol | propylene oxide/8 | $NH_3CH_2CH_2OH$ | 77.1 | 69.5 |
| 16 | N-Octanol | propylene oxide/8 | $NH_3CH_2CH(CH_3)OH$ | 81.3 | 78.7 |
| 17 | Neodol 25 | ethylene oxide/3 | Na | 58.7 | 55.6 |

EXAMPLES 18 to 23

A number of surfactants of the invention of the kind wherein R is a branched hydrocarbon radical were prepared using the synthetic procedures outlined in paragraphs A and B-1 above. Their properties are set forth in Table IV below.

TABLE IV

Properties of Alkoxylated Alcohol Sodium Sulfates

| Example No. | Alcohol | Alkylene Oxide/ Units | Surface Tension(2) (Dynes/cm) | Draves Wetting(3) (Sec) | Ross Miles Foaming (cm)(4) Initial | Ross Miles Foaming (cm)(4) 5 Minutes | Detergency(5) Polyester % SR | Detergency(5) Polyester % D |
|---|---|---|---|---|---|---|---|---|
| 18 | 2-Ethyl-hexanol | Propylene oxide/ 5 | 31.9 | 16.0 | -- | -- | 69.4 | 52.7 |
| 19 | 2-Ethyl-hexanol | Propylene oxide/ 8 | 32.5 | 17.1 | 19.1 | 2.1 | 68.5 | 61.9 |
| 20 | 2-Ethyl-hexanol | Propylene oxide/ 10 | 35.1 | 22.2 | 18.3 | 1.0 | 74.2 | 73.7 |
| 21 | 2-Ethyl-hexanol | Propylene oxide/ 12 | 36.2 | 21.2 | -- | -- | 72.9 | 70.1 |
| 22 | 2-Ethyl-hexanol | Ethylene oxide/ 5 | 42.5 | >1800 | -- | -- | 0.0 | 0.0 |
| 23 | Neodol®25(1) | Ethylene oxide/ 3 | 34.8 | 17.8 | 20.0 | 20.0 | 73.9 | 70.4 |

Notes:

(1) Neodol®25 = Shell $C_{12-15}$ fatty alcohol
(2) Surface Tension is measured on a 1% solution at 25°C; Kraft Points is 1°.
(3) Draves wetting is measured on a 0.1 wt% solution at room temperature (77°F)
(4) Ross Miles Foaming procedure outlined in ASTM D1173-53 was used.
(5) Terg-o-tometer test with: Speed = 125 rpms; Temperature = 120°F; Time = 10 minutes/ Surfactant concentration = .15 wt%; hardness = 150 ppm; 4" x 6" cloth - each wash batch used 5 soiled and 3 clean polyester fabric; the panels used in determining evalutation were coated with standard pad applied airborne/dust sebum soil.

TABLE IV (continued)

$$\text{\% Soil Removal (SR)} = \frac{R_L \text{ Soil BW} \times 100}{R_L \text{ Unsoiled} - R_L \text{ Soil BW}}$$

$$\text{\% Detergency (D)} = \frac{R_L \text{ Soil AW} - (R_L \text{ Redeposition BW-AW})}{R_L \text{ Redeposition} - R_L \text{ Soil BW}} \times$$

$R_L$ = Reflectance (L Scale)

AW = After Wash

BW = Before Wash

The results set forth in Table IV demonstrate the obtainment of improved results of the composition of the present invention (Examples 18-21) with regard to wetting, foaming and as a detergent with respect to polyester fabric as compared with an ethoxylated surfactant (Example 22) and the obtainment of properties at least equal to a commercially available surfactant (Example 23).

Examples 24 to 27

The experimental procedure outlined under Example Nos. 18-21 was followed to prepare an alkoxylated anionic derivative of the invention (Example 24), which was compared with various commercially available detergents. The results are set forth in Table V, below, the detergency results having been obtained in the same manner as in Table IV, above.

TABLE V

Detergency on Polyester of Propylated 2-Ethylhexanol Sodium Sulfate vs. Commercial Surfactants

| Example No. | Surfactant | Concentration (% Active) | Detergency Polyester %SR | %D |
|---|---|---|---|---|
| 24 | 2-Ethylhexanol $(PO)_{10}$ $SO_3Na+$ | 85.2 | 78.9 | 75.6 |
| 25 | Neodol® 25-3S(1) | 58.9 | 74.8 | 72.8 |
| 26 | UltraWet® 60K Dodecylbenzene Sulfonate | 64.0 | 62.3 | 62.3 |
| 27 | ERA® (Neodol® 25-7; Nonionic)(1) | 33.7 | 72.4 | 70.6 |

(1) Ethoxylated $C_{12}$-$C_{15}$ fatty alcohol

The results set forth above in Table V demonstrate detergency of the surfactant of the invention to be equal to or better than existing commercial products and that the compositions of the invention can be supplied with substantially higher concentrations of active ingredients as compared with commercial compositions of Examples 25 to 27, inclusive.

It can thus be seen from Examples 1 to 27 that valuable and highly desirable alkoxylated ether sulfate anionic surfactants which exhibit excellent detergency, high activity, good wetting, low foaming and fabric softening properties have been provided.

EXAMPLES 28 to 47

A number of admixture surfactants of the invention were prepared using the synthetic procedures outlined in paragraphs A and B-1 above. Their properties are set forth in Table VI below.

TABLE VI

Properties of Alkoxylated Alcohol Sodium Sulfates and Other Surfactants

| Example No. | Alcohol or Surfactant | Alkylene Oxide/Units | Draves Wetting[6] (Sec) | Surface Tension[7] (Dynes/cm) | Concentration (% Active) | Viscosity (cps) | Detergency[8] Polyester % SR | % D |
|---|---|---|---|---|---|---|---|---|
| 28 | Epal* 108 | propylene oxide/5 | 8.5 | 30.4 | 92.1 | 402 | 58.6 | 40.4 |
| 29 | Epal* 810[2] | propylene oxide/5 | 11.7 | 33.3 | 81.4 | 376 | 68.6 | 64.8 |
| 30 | Epal* 610[3] | propylene oxide/5 | 10.6 | 35.0 | 90.2 | 245 | 85.9 | 82.7 |
| 31 | Epal* 610 | propylene oxide/8 | -- | -- | 90.2 | -- | 85.9 | 82.7 |
| 32 | Epal* 810 | propylene oxide/8 | -- | -- | 90.0 | -- | 90.0 | 90.6 |
| 33 | Epal* 108 | propylene oxide/8 | -- | -- | 98.4 | -- | 82.4 | 78.0 |
| 34 | Epal* 810 | propylene oxide/12 | -- | -- | 96.2 | -- | 83.3 | 81.7 |
| 35 | Epal* 108 | propylene oxide/10 | -- | -- | 94.3 | -- | 83.8 | 83.8 |
| 36 | Epal* 108 | propylene oxide/12 | -- | -- | 92.7 | -- | 84.1 | 81.5 |
| 37 | Epal* 108 | propylene oxide/8[9] | -- | -- | 91.7 | -- | 78.9 | 77.2 |
| 38 | Iso-$C_9$ "oxo" mixture | propylene oxide/5 | 10.0 | 34.6 | 93.6 | -- | 75.4 | 61.9 |
| 39 | Iso-$C_9$ "oxo" mixture | propylene oxide/10 | 5.7 | 34.1 | 92.8 | -- | 73.9 | 71.3 |
| 40 | Epal* 108[1] | ethylene oxide/5 | >1200 | 38.1 | 73.5 | 257 | 0 | 0 |
| 41 | 2-Ethylhexanol | ethylene oxide/5 | >1800 | 42.5 | -- | -- | 0 | 0 |
| 42 | n-Octanol | propylene oxide/8 | 31.0 | 34.2 | -- | -- | 75.0 | 68.3 |

TABLE VI (continued)

Properties of Alkoxylated Alcohol Sodium Sulfates and Other Surfactants

| Example No. | Alcohol or Surfactant | Alkylene Oxide/units | Draves Wetting[6] (Sec) | Surface Tension[7] (Dynes/cm) | Concentration (% Active) | Viscosity (cps) | Detergency[8] Polyester % SR | % D |
|---|---|---|---|---|---|---|---|---|
| 43 | n-Heptanol | propylene oxide/8 | 29.2 | 39.2 | -- | -- | 53.1 | 50.1 |
| 44 | n-Octanol | propylene oxide/5 | 210.0 | 38.1 | -- | -- | 66.2 | 41.9 |
| 45 | Neodol 25-3S Ethoxylated Surfactant[4] | -- | 18.0 | 34.2 | 58.9 | -- | 74.8 | 72.8 |
| 46 | UltraWet* 60K Dodecyl-benzene Sulfonate | -- | -- | -- | 64.0 | -- | 62.3 | 62.3 |
| 47 | Neodol 25-7 Non-Ionic Surfactant[5] | -- | -- | -- | 33.7 | -- | 72.4 | 70.6 |

Notes:

(1) A "plasticiser" grade alcohol comprised of about 56 percent octanol, 42.9 percent decanol, 0.3 percent hexanol and 0.7 percent dodecanol.

(2) A "plasticiser" grade alcohol comprised of about 44.3 percent octanol, 54.5 percent decanol and 0.3 percent hexanol.

(3) A "plasticiser" grade alcohol comprised of about 40.0 percent octanol, 55.4 percent decanol, 3.6 percent hexanol and 0.9 percent dodecanol.

(4) An anionic sulfate surfactant derived from the ethoxylation with three units of ethylene oxide of a detergent grade alcohol comprised of $C_{12}$-$C_{15}$ carbon atom aliphatic alcohol.

* Registered Trade Mark

(5) A nonionic surfactant derived from detergent grade $C_{12-15}$ alcohol.

(6) Draves wetting is measured on a 0.1 wt% solution at room temperature (77°F).

(7) Surface Tension is measured on a 1% solution at 25°C; Kraft Points is 1°.

(8) Terg-o-tometer test with: Speed = 125 rpms; Temperature = 120°F; Time = 10 minutes/ Surfactant concentration = .15 wt hardness = 150 ppm; 4" x 6" cloth - each wash batch used 5 soiled and 3 clean polyester fabric; the panels used in determining evalutation were coated with standard pad applied airborne/dust sebum soil.

$$\text{\% Soil Removal (SR)} = \frac{R_L \text{ Soil BW} \times 100}{R_L \text{ Unsoiled} - R_L \text{ Soil BW}}$$

$$\text{\% Detergency (D)} = \frac{R_L \text{ Soil Aw} - (R_L \text{ Redeposition BW-AW}) \times}{R_L \text{ Redeposition} - R_L \text{ Soil BW}}$$

$R_L$ = Reflectance (L Scale)

AW = After Wash

BW = Before Wash

(9) The propoxylated surfactant obtained by propoxylation of Epal® 108 alcohol was end-capped with three units of ethylene oxide.

The results set forth in Table VI comparing typical surfactant admixture compositions of the present invention (Examples 28-39) with commercially available surfactants (Examples 45-47), as well as with a number of surfactants derived from ethoxylation of specific alcohol substrates or surfactants derived from propoxylation of individual alcohols (Examples 38-44), demonstrate the obtainment of at least equal or improved surface activity, wetting, and detergency with respect to polyester fabric. These results additionally show the obtainment of highly concentrated, i.e. above 90% active, liquid, non-viscous compositions of the invention as compared with commercially

available compositions of substantially lesser activity.

EXAMPLES 48 to 54.

The experimental procedure outlined under Examples 28-44 was followed to prepare alkoxylated derivative admixtures of the invention, while varying the base employed for neutralization, to demonstrate the counter-ion effect upon surface active properties;and surface tension, Draves wetting and detergency were obtained in the same manner as in Table VI above. The results are set forth in Table VII below.

TABLE VII

| Example No. | Alcohol/ Surfactant | Alkylene Oxide/ Units | Counter-ion(M)$^+$ | Draves Wetting (Sec) | Surface Tension (Dynes/cm) | Detergency Polyester % SR | %D |
|---|---|---|---|---|---|---|---|
| 48 | Iso-$C_9$ "OXO" Mixture | propylene oxide/ 5 | $^+Na$ | 10.0 | 34.6 | 75.4 | 69.7 |
| 49 | Iso-$C_9$ "OXO" Mixture | propylene oxide/ 5 | $^+K$ | 9.3 | 31.3 | 84.6 | 81.3 |
| 50 | Iso-$C_9$ "OXO" Mixture | propylene oxide/ 5 | $^+NH_4$ | 13.0 | 30.6 | 80.5 | 77.9 |
| 51 | Iso-$C_9$ "OXO" Mixture | propylene oxide/ 5 | $^+NH_3CH_2CH_2OH$ | 22.0 | 33.8 | 79.1 | 69.7 |
| 52 | Iso-$C_9$ "OXO" Mixture | propylene oxide/ 5 | $^+NH_3CH_2CHOH$ | 29.0 | 34.9 | 75.8 | 75.3 |
| 53 | Iso-$C_9$ "OXO" Mixture | butylene oxide/5 | $^+Na$ | 5.8 | 30.2 | 81.5 | 78.5 |
| 54 | Neodol 25-3S(1) | | $^+Na$ | 18.0 | 34.6 | 71.0 | 68.4 |

(1) Commercially available ethoxylated $C_{12}$-$C_{15}$ fatty alcohol

It can thus be seen from Examples 28 to 54 above that valuable and highly desirable alkoxylated ether sulfate anionic surfactant admixtures which exhibit excellent detergency, viscosity characteristics, wetting capabilities, hard water stability, and surface activity have been provided. Further, these compositions are characterised as being of high active concentration, and may be readily diluted with water without formation of gels, lumps and highly viscous compositions of low flowability.

Although the alkoxylated ether sulfate anionic surfactants described in the foregoing have useful detergent properties per se, it is generally preferred to use them in combination with other detergent active compounds and with various adjuvants such as hydrotopes, typically cumene, xylene, and toluene sulfonates, perfumes, pH modifiers, inorganic salts, bacteriastats, dyes, solvents such as alkanols and carbitols, typically ethanol, isopropanol, methyl carbitol and ethyl carbitol and the like. Further, if desired, the surfactant compositions of the invention may be employed with other adjuvants specific to desired applications such as carboxymethyl cellulose, optical brighteners, corrosion inhibitors, alkaline builders, and the like, as is well known in the art.

Our invention has been described and illustrated by reference to specific embodiments thereof, and the examples illustrate the best mode presently known for carrying out the invention. It should be noted, however, that variations of these procedures are feasible and many such variations would be obvious to those skilled in the art in view of the disclosures contained herein.

CLAIMS

1. A water-soluble, liquid alkoxylated ether sulfate anionic surfactant having the formula:

$RO[CH_2CH(R')O]_m Z_n SO_3M$ wherein

R is a primary hydrocarbon linear alkyl radical containing of from about 4 to 10 carbon atoms or a branched chain hydrocarbon alkyl radical containing from about 4 to 11 carbon atoms; R' is a member selected from the group consisting of methyl and ethyl; m is an integer which is from 7 to 12 when R is a primary hydrocarbon radical and from 5 to 12 when R is a branched chain hydrocarbon radical; Z is an oxyethylene group or a random mixture of oxyethylene groups and oxyalkylene groups present in the radical $-[CH_2CH(R')O]-$, the molar ratio of oxyethylene to oxyalkylene groups in said mixture being such that the total molar ratio of oxyethylene to oxyalkylene groups in said formula is from about 1:1 to 1:10; n is an integer of from 0 to 4; and M is hydrogen, an alkali metal, an alkaline earth metal; ammonium or a primary, secondary, tertiary or quaternary alkyl ammonium or alkylolammonium ion.

2. The anionic surfactant of claim 1 wherein R is an alkyl radical containing of from 8 to 10 carbon atoms.

3. The anionic surfactant of claim 1 or claim 2 wherein R' is methyl.

4. The anionic surfactant of any one of claims 1 to 3 wherein M is selected from alkali metals, ammonium and hydrogen.

5. The anionic surfactant of claim 4 wherein M is sodium or potassium.

6. The anionic surfactant of claim 3 wherein m is an integer of from 8 to 10; n is 0 and M is sodium.

7. A water-soluble, liquid admixture of alkoxylated ether sulfate anionic surfactants corresponding to the formula:

$RO[CH_2CH(R')O]_mZ_nSO_3M$ wherein

R is a straight or branched hydrocarbon alkyl radical containing of from about 4 to 11 carbon atoms; R' is a member selected from the group consisting of methyl and ethyl; m is an integer of from 5 to 12; Z is an oxyethylene group or a random mixture of oxyethylene groups and oxyalkylene groups present in the radical $-[CH_2CH(R')O]-$, the molar ratio of oxyethylene to oxyalkylene groups in said mixture being such that the total molar ratio of oxyethylene to oxyalkylene groups in said formula is from about 1:1 to 1:10; n is an integer of from 0 to 4; and M is hydrogen, an alkali metal, an alkaline earth metal; ammonium or a primary, secondary, tertiary or quaternary alkyl ammonium or alkylolammonium ion, said admixture being characterised as containing at least two of said surfactants present within a molar ratio of between about 4:1 and 1:4.

8. The anionic surfactant admixture of claim 7 wherein R' is methyl.

9. The anionic surfactant admixture of claim 7 or claim 8 wherein R is an alkyl radical containing of from 6 to 10 carbon atoms.

10. The anionic surfactant admixture of claim 9 wherein M is selected from alkali metals, ammonium and hydrogen.

11. The anionic surfactant admixture of claim 11 wherein M is sodium or potassium.

12. The anionic surfactant of claim 8 wherein m is an integer of from 6 to 10; n is 0 and M is sodium.

13. The anionic surfactant admixture of claim 12 wherein the admixture is further characterised as being obtained by (i) propoxylation of a substantially straight chain alcohol admixture comprising between about 40 and 60 percent, by weight, of octanol, 40 and 60 percent, by weight, of decanol, and up to about 5 percent, by weight, of hexanol; (ii) sulfation with a member selected from the group consisting of chlorosulfonic acid and sulfur trioxide; and (iii) neutralisation with

sodium hydroxide.

14. The anionic surfactant admixture of claim 10 wherein the admixture is further characterised as being obtained by (i) propoxylation of a substantially branched chain admixture selected from the group consisting of isononyl alcohols and 2-ethyl hexanol; (ii) sulfation with a member selected from the group consisting of chlorosulfonic acid and sulfur trioxide; and (iii) neutralisation with sodium hydroxide.

15. An aqueous, liquid surfactant concentrate composition comprising at least about 5%, but not more than 50%, by weight, of water, and an active anionic surfactant as claimed in any one of claims 1 to 14.